# EUROPEAN PATENT SPECIFICATION

(11) **EP 4 087 508 B1**
(45) Date of publication and mention of the grant of the patent: **18.12.2024**
(21) Application number: 21835412.4
(22) Date of filing: 20.12.2021
(51) Int. Cl.: A61B 18/14, A61B 34/00, B29C 45/00

(54) **SEAL FOR SURGICAL INSTRUMENT**
DICHTUNG FÜR CHIRURGISCHES INSTRUMENT
JOINT POUR INSTRUMENT CHIRURGICAL

(30) Priority: 21.12.2020 US 202017129013
(43) Date of publication of application: 16.11.2022
(73) Proprietor: Cilag GmbH International, 6300 Zug (CH)
(72) Inventor: MUMAW, Daniel J., Cincinnati, Ohio 45242 (US); BURCHNALL, Mark R., Mason, Ohio 45040 (US); KLOPPENBORG, Nicholas M., Cincinnati, Ohio 45236 (US); MAXWELL, Michael B., Cincinnati, Ohio 45208 (US); KURTH, David W., Butler, Wisconsin 53007 (US)
(74) Representative: Carpmaels & Ransford LLP
(86) International application number: PCT/IB2021/062030
(87) International publication number: WO 2022/137086

(56) References cited:
- EP-A2- 2 260 782
- DE-A1- 2 748 057
- US-A1- 2010 016 852
- US-A1- 2020 315 722

## Description

### BACKGROUND

A variety of surgical instruments include an end effector for use in conventional medical treatments and procedures conducted by a medical professional operator, as well as applications in robotically assisted surgeries. Such surgical instruments may be directly gripped and manipulated by a surgeon or incorporated into robotically assisted surgery. In the case of robotically assisted surgery, the surgeon may operate a master controller to remotely control the motion of such surgical instruments at a surgical site. The controller may be separated from the patient by a significant distance (e.g., across the operating room, in a different room, or in a completely different building than the patient). Alternatively, a controller may be positioned quite near the patient in the operating room. Regardless, the controller may include one or more hand input devices (such as joysticks, exoskeletal gloves, master manipulators, or the like), which are coupled by a servo mechanism to the surgical instrument. In one example, a servo motor moves a manipulator supporting the surgical instrument based on the surgeon's manipulation of the hand input devices. During the surgery, the surgeon may employ, via a robotic surgical system, a variety of surgical instruments including an ultrasonic blade, radio frequency tissue cutters and scissors, a tissue grasper, a needle driver, an electrosurgical cautery probes, etc. Each of these structures performs functions for the surgeon, for example, cutting tissue, coagulating tissue, holding or driving a needle, grasping a blood vessel, dissecting tissue, or cauterizing tissue.

In one example, employed surgical instruments are operable to cut and/or seal tissue by applying radiofrequency (RF) electrosurgical energy to the tissue. Examples of such devices and related concepts are disclosed in U.S. Pat. No. 7,354,440, entitled "Electrosurgical Instrument and Method of Use," issued April 8, 2008, the disclosure of which is referred to herein; and U.S. Pat. No. 7,381,209, entitled "Electrosurgical Instrument," issued June 3, 2008, the disclosure of which is referred to herein.

Document US 2010/016852 A1 discloses an electrically energized medical instrument that uses one or more drive cables to both actuate mechanical components of a wrist mechanism or an effector and to electrically energize the effector. Electrical isolation can be achieved by using an insulating main tube through which the drive cables extend from a backend mechanism to the effector, with an insulating end cover that leaves only the desired portions of the effector exposed, and one or more seals to prevent electrically conductive liquid from entering the main tube.

Document US 2020/315722 A1 an electrosurgical instrument having jaws energized and actuated using a length of cable. The cable is formed of a conductive inner portion coated with a dielectric polymer. The cable extends through a pass-through in a portion of the jaw and is crimped to create a mechanical and electrical connection between the conductive inner portion and the conductive jaw material. the cables exit the shaft via a seal that includes passages for each of the cables. This seal functions as an assembly tool during the assembly process, by helping align the cables. During the final stages of assembly, it is compressed longitudinally to create radial seals against the inner diameter of the outer shaft and the outer diameter of the inner shaft.

While various kinds of surgical instruments and associated components have been made and used, it is believed that no one prior to the inventor(s) has made or used the invention described in the appended claims.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts perspective view of an exemplary electrosurgical tool in combination with a generator, shown schematically;
FIG. 2 depicts a perspective view of a distal portion of another exemplary electrosurgical tool;
FIG. 3 depicts another perspective view of a distal portion of the tool of FIG. 2;
FIG. 4 depicts a side view of an intermediate portion of the tool of FIG. 2;
FIG. 5 depicts yet another perspective view of a distal portion of the tool of FIG. 2;
FIG. 6 depicts a perspective view of an exemplary proximal clevis that may be readily incorporated into the tool of FIG. 2, showing the proximal clevis coupled to a distal shaft portion of the tool of FIG. 2;
FIG. 7 depicts a perspective cross-sectional view of the proximal clevis and distal shaft portion of FIG. 6, the cross-section taken along line 7-7 of FIG. 6;
FIG. 8 depicts another perspective cross-sectional view of the proximal clevis and distal shaft portion of FIG. 6, the cross-section taken along line 8-8 of FIG. 6;
FIG. 9 depicts a perspective view of a seal feature of the proximal clevis of FIG. 6;
FIG. 10 depicts another perspective view of the seal feature of FIG. 9;
FIG. 11A depicts a detailed perspective view of a portion of the seal feature of FIG. 9, with a web in position during an overmolding process;
FIG. 11B depicts another detailed perspective view of a portion of the seal feature of FIG. 9, with the web of FIG. 11A removed;
FIG. 12 depicts a schematic view of an exemplary mold for use with the overmolding process of FIG. 11A;
FIG. 13 depicts yet another perspective cross-sectional view of the proximal clevis and distal shaft portion of FIG. 6, with cables and a wire of the tool of FIG. 2 extending therethrough;
FIG. 14 depicts still another perspective cross-sectional view of the proximal clevis and distal shaft portion of FIG. 6, with the cables and the wire of FIG. 12 extending therethrough;
FIG. 15 depicts an enlarged side cross-sectional view of the proximal clevis and distal shaft portion of FIG. 6, with the cables and the wire of FIG. 12 extending therethrough;
FIG. 16 depicts a perspective view of another exemplary proximal clevis that may be readily incorporated into the tool of FIG. 2, showing the proximal clevis coupled to a distal shaft portion of the tool of FIG. 2;
FIG. 17 depicts a perspective cross-sectional view of the proximal clevis and distal shaft portion of FIG. 16, the cross-section taken along line 17-17 of FIG. 16;

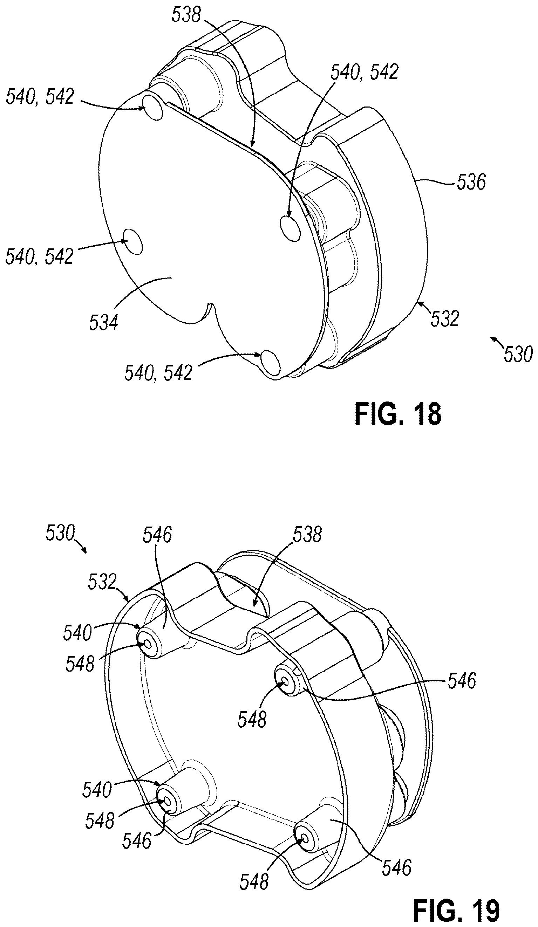

FIG. 18 depicts a perspective view of a seal feature of the proximal clevis of FIG. 16;
FIG. 19 depicts another perspective view of the seal feature of FIG. 18; and
FIG. 20 depicts another perspective cross-sectional view of the proximal clevis and distal shaft portion of FIG. 16, with cables of the tool of FIG. 2 extending therethrough.

The drawings are not intended to be limiting in any way, and it is contemplated that various embodiments may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown, but is defined in the appended claims.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "lateral," "transverse," "bottom," "top," are relative terms to provide additional clarity to the figure descriptions provided below.

### I. Exemplary Electrosurgical Tool

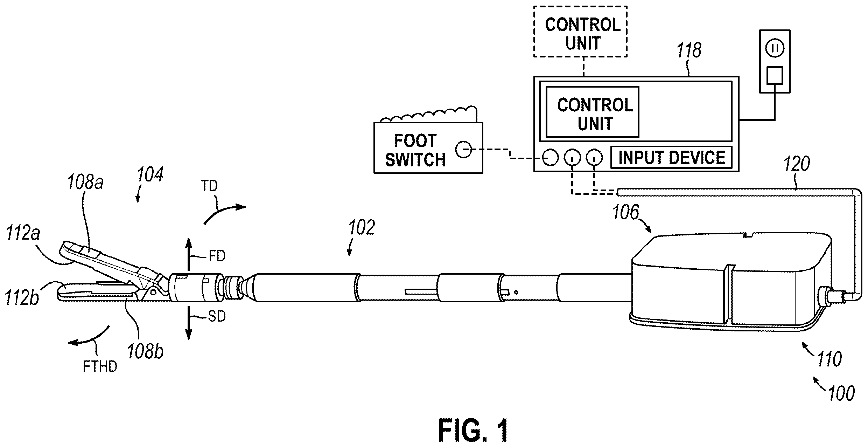

FIG. 1 shows an exemplary electrosurgical tool (100). Although the term "tool" is used herein in connection with electrosurgical tool (100), it should be understood that in other contexts, the term "instrument" may alternatively be used. Tool (100) includes an elongate shaft (102), an end effector (104) coupled to a distal end of the shaft (102), and a proximal housing portion (106) including a housing (110) coupled to a proximal end of shaft (102). End effector (104) in the present example includes first and second jaw members (108a, 108b), also referred to herein as "jaws," and is configured to move between an open configuration and a closed configuration. End effector (104) is shown in the open configuration in FIG. 1. First and second jaw members (108a, 108b) are generally of a straight configuration, but in other examples one or both of first and second jaw members (108a, 108b) can be of a curved configuration. Jaw members (108a, 108b) are configured to close to thereby capture or engage tissue so as to clamp or grasp the tissue therebetween. Thus, it should be understood that first and second jaw members (108a, 108b) are generally configured to apply compression to clamped tissue.

One or both of jaw members (108a, 108b) may include an electrode for providing electrosurgical energy to tissue. By way of example only, each of first and second jaw members (108a, 108b) may include at least one electrode, e.g., tool (100) is bipolar, such that electrical current can flow between the electrodes in the opposing jaw members (108a, 108b) and through tissue positioned therebetween. In the present example, first jaw member (108a) has an electrode (112a) on a tissue-facing surface thereof and second jaw member (108b) has an electrode (112b) on a tissue-facing surface thereof. Although jaw members (108a, 108b) of the present example are configured with a bipolar electrode configuration, it should be understood that in other examples, jaw members (108a, 108b) may be configured with a monopolar configuration where only one jaw member (108 a, 108 b) of jaw members (108a, 108b) includes an electrode (112a, 112b). Electrodes (112a, 112b) are each generally configured to be positioned against and/or positioned relative to tissue such that electrical current can flow through the tissue. The electrical current may generate heat in the tissue that, in turn, causes one or more hemostatic seals to form within the tissue and/or between tissues. For example, tissue heating caused by the electrical current may at least partially denature proteins within the tissue. Such proteins, such as collagen, may be denatured into a proteinaceous amalgam that intermixes and fuses, or "coagulates" or "welds," together as the proteins renature. As the treated region heals over time, this biological "weld" may be reabsorbed by the body's wound healing process. As described above, the energy applied can include high frequency alternating current such as RF energy. When applied to tissue, RF energy may cause ionic agitation or friction, increasing the temperature of the tissue. Various embodiments of applying RF energy are described further in U.S. Patent No. 10,441,345 entitled "Surgical Generator For Ultrasonic And Electrosurgical Devices" issued Oct. 15, 2019, U.S. Patent No. 9,161,803 entitled "Motor Driven Electrosurgical Device With Mechanical And Electrical Feedback" issued Oct. 20, 2015, and U.S. Patent Publication No. 9,802,033 entitled "Surgical Devices Having Controlled Tissue Cutting And Sealing" issued Oct. 31, 2017, which are hereby referred to in their entireties.

Although not shown, it should be understood that in some examples tool (100) may include a cutting element (not shown), which can be configured as a knife on an I-beam or other suitable structure. Suitable cutting elements may be configured to translate along the axial length of end effector (104), to thereby cut or transect tissue positioned between jaws members (108a, 108b). Such cutting may occur during or after the application of electrosurgical energy.

Tool (100) is configured to operatively couple with a generator (118). Tool (100) is connected to generator (118) with a cable (120) in the present example, but can connect thereto in other ways, as will be appreciated by a person skilled in the art. Generator (118) is configured as an energy source, e.g., an RF source, an ultrasonic source, a direct current source, etc., to deliver energy to tool (100) to permit electrodes (112 a, 112 b) to apply energy to tissue. In the present example, generator (118) may be coupled to a controller, such as a control unit. The control unit may be formed integrally with generator (118) or may be provided as a separate and independent device electrically coupled to generator (118) (shown in phantom in FIG. 1 to illustrate this option). A suitable control unit may be configured to regulate the energy delivered by generator (118) which in turn delivers energy to electrodes (112 a, 112 b). The energy delivery may be initiated in any suitable manner. By way of example only, tool (100) may be energized by generator (118) via actuation of a foot switch. When actuated, the foot switch (or other actuated actuator) triggers generator (118) to deliver energy to end effector (104). The control unit can be configured to regulate the power generated by generator (118), as discussed for example further below. As also discussed further below, the control unit as a separate and independent device from generator (118) can be part of a robotic surgical system.

Proximal housing portion (106), e.g., within housing (110), includes a drive system (not shown) configured to operably couple to at least one motor for driving the drive system to cause performance of various functions of tool (100), such as closing of jaw members (108a, 108b), opening of jaw members (108a, 108b), articulating end effector (104) relative to shaft (102), rotating shaft (102) about a longitudinal axis thereof, movement of the cutting element (not shown) along end effector (104), and application of energy. By way of example only, tool (100) may include a drive system having one or more separate drive systems configured to drive various components of tool (100). For instance, the drive system may include separate or combined drive assemblies configured to drive rotation of shaft (102), drive rotation of end defector, drive articulation of end effector (104) in opposed first and second directions (FD, SD), drive articulation of end effector (104) in opposed third and fourth directions (TD, FTHD), drive a closure assembly to selectively cause opening and closing of end effector (104), and/or etc. Although not shown in specific detail, it should be understood that each drive system may include one or more mechanical or electromechanical components operable to drive the various movements of shaft (102) and/or end effector (104) described above. Additionally, one or more of each drive system can be in communication with another drive system to, for example, drive multiple movements using a single rotary input. By way of example only, suitable mechanical or electromechanical components may include gears, cables, springs, belts, lead screws, splines, linear actuators, cylinders, pistons, racks, pinions, and/or etc. In some examples, each drive system can be configured in accordance with at least some of the teachings of U.S. Patent Publication No. 2019/0059987 entitled "Methods, Systems, and Devices for Controlling Electrosurgical Tools" filed Aug. 29, 2017, the disclosure of which is referred to herein in entirety.

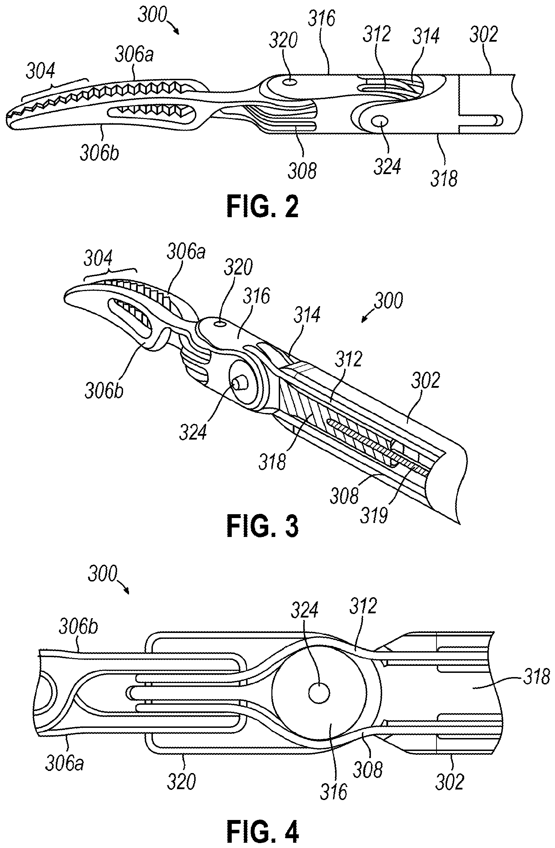

FIGS. 2-5 show an exemplary alternative electrosurgical tool (300) that is substantially similar to tool (100) described above unless otherwise noted herein. For instance, tool (300) of the present example is generally configured and used similar to tool (100) of FIG. 1 and likewise includes an elongate shaft (302), an end effector (304) coupled to a distal end of shaft (302) and including first and second jaw members (306 a, 306 b), and a proximal housing portion (not shown) including a drive system (not shown) coupled to a proximal end of shaft (302). One or both jaw members (306a, 306b) may include an electrode operable to deliver RF energy to tissue. Similar to proximal housing portion (106) of FIG. 1 discussed above, the proximal housing portion of tool (300) may be configured to operably couple to a tool driver of a robotic surgical system, or the proximal housing portion can be configured to be handheld and operated manually.

Tool (300) includes a plurality of elongate actuatable drive members shown in the form of cables (308, 310, 312, 314) that are configured to be actuated to selectively cause opening of end effector (304), closing of end effector (304), and articulation of end effector (304) relative to shaft (302). Cables (308, 310, 312, 314) are attached to end effector (304) and extend along solid surfaces of guide channels in end effector (304), a distal clevis (316), and a proximal clevis (318), and from there extend proximally through shaft (302) to the proximal housing portion.

In addition to cables (308, 310, 312, 314), tool (300) may also include one or more elongate conductive members shown in the form of wires (319) extending through shaft (302) to end effector (304) or other portions of tool (300). For instance, as best seen in FIG. 3, one or more wires (319) may extend through shaft (302), proximal clevis (318), and distal clevis (316) to end effector (304). Such one or more wires (319) may be used for a variety of conductive purposes. For instance, as noted above, tool (300) configured to operatively connect to a generator substantially similar to generator (118) for generating energy that can be used in connection with end effector (304). By way of example only, in some configurations end effector (304) can be equipped with electrodes similar to electrodes (112 a, 112 b) described above. In such examples, one or more wires (319) may be used to supply RF energy from the generator to the electrodes. Of course, in other examples, various alternative uses for one or more wires (319) may be used as will be apparent to those of ordinary skill in the art in view of the teachings herein.

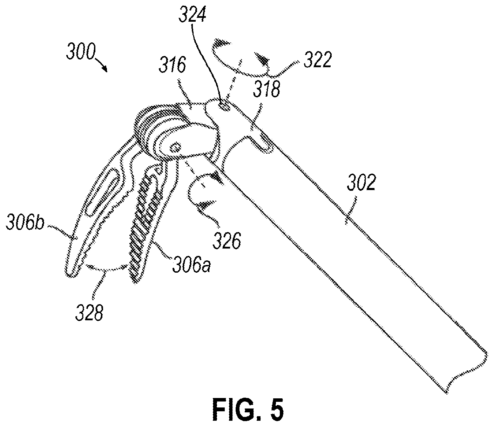

As best seen in FIG. 5, distal clevis (316) is configured to rotate (322) relative to proximal clevis (318) about a pin (324) that defines a pitch axis. Rotation of distal clevis (316) relative to proximal clevis (318) is driven about the pitch axis in response to cable actuation. In particular, for clockwise rotation about the pitch axis, a drive system in response to control thereof (e.g., in response to motor force delivered thereto) pulls in identical lengths of third and fourth cables (312, 314) while releasing the same lengths of first and second cables (308, 310). Third and fourth cables (312, 314) apply forces to distal clevis (316) at moment arms defined by guide channels of third and fourth cables (312, 314) through distal clevis (316). Similarly, for counterclockwise rotation of distal clevis (316) about the pitch axis, the drive system in response to control thereof pulls in identical lengths of first and second cables (308, 310) while releasing the same lengths of third and fourth cables (312, 314).

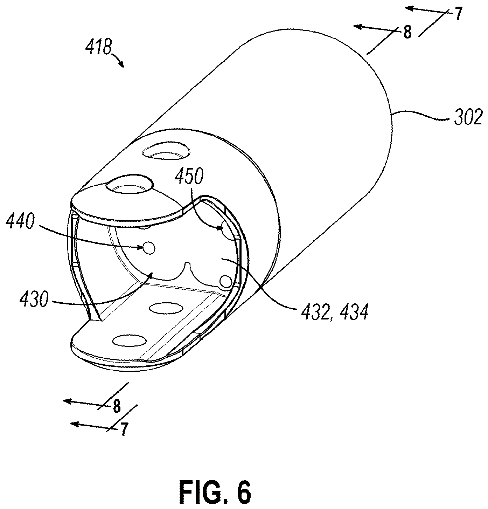

Proximal clevis (318) is fastened to the distal end of shaft (302) and extends distally therefrom. Proximal clevis (318) is generally configured to receive a portion of distal clevis (316) to permit rotation (322) of distal clevis (316) relative to shaft (302). In other words, proximal clevis (318) provides a point of connection between distal clevis (316) and shaft (302) to promote movement of distal clevis (316) relative to shaft (302). As noted above, distal clevis (316) is fastened to proximal clevis (318) by pin (324), which defines the pitch axis. Additionally, as best seen in FIG. 6, proximal clevis (318) is generally hollow such that cables (308, 310, 312, 314) and/or one or more wires (319) may extend through proximal clevis (318) to distal clevis (316) and/or end effector (304).

A pin (320) in distal clevis (316) is perpendicular to pin (324) and defines a pivot or yaw axis, about which end effector (304) is configured to rotate (326) relative to distal clevis (316) and about which jaw members (306 a, 306 b) are configured to individually rotate (328) to open and close in response to cable actuation. In particular, first and second cables (308, 310) attach to first jaw member (306 a), and third and fourth cables (312, 314) attach to second jaw member (306 b). The attachment of first and second cables (308, 310) to first jaw member (306 a) is such that pulling in a length of one cable (308 or 310) while releasing the same length of the other cable (308 or 310) causes first jaw member (306 a) to rotate about pin (320). Similarly, the attachment of third and fourth cables (312, 314) to second jaw member (306 b) is such that pulling in a length of one cable (312 or 314) while releasing the same length of the other cable (312 or 314) causes second jaw member (306 b) to rotate about pin (320). A closure assembly of tool (300) thus includes cables (308, 310, 312, 314).

Cables (308, 310, 312, 314) may be driven by one or more drive systems configured to selectively pull cables (308, 310, 312, 314) in proximal directions. It will be appreciated that such drive systems and other features and functions of surgical tool (300) may be configured in accordance with at least some of the teachings of U.S. Patent Publication No. 2019/0059987. Exemplary embodiments of electrosurgical tools are further described in U.S. Pat. No. 9,119,657 entitled "Rotary Actuatable Closure Arrangement For Surgical End Effector" filed Jun. 28, 2012 and U.S. Pat. No. 8,771,270 entitled "Bipolar Cautery Instrument" filed Jul. 16, 2008, which are hereby referred to herein in their entireties.

### II. Exemplary Alternative Clevis having Sealing Feature

In some instances, it may be desirable to equip portions of electrosurgical tools (100, 300) with sealing components. Such sealing components may be desirable to prevent fluid ingress into the tools (100, 300) and to maintain insufflation of a patient's body cavity during a laparoscopic surgical procedure. Fluid ingress is generally undesirable in both robotic and hand-held applications of the instrument. For instance, fluid ingress in certain shaft portions of a surgical instrument can be particularly challenging to effectively clean and/or sterilize between surgical procedures. Moreover, fluid ingress can be indicative of an ineffectively sealed structure that is thus prone to leaking insufflation gas from an insufflated body cavity, which can reduce the efficiency of a surgical procedure.

Accordingly, it is generally desirable to incorporate robust sealing components into various portions of electrosurgical tools (100, 300). Various examples of sealing components will be described in greater detail below. However, it should be understood that various alternative configurations may be used without departing from the nature of the subject matter described herein. Additionally, although sealing components described herein are described in connection with electrosurgical tools (100, 300), it should be understood that such sealing components may be readily incorporated into various alternative surgical tools different from those described herein, such as surgical tools configured to grasp, cut, and/or staple tissue, where such tools may or may not be configured to apply RF energy to tissue.

FIG. 6 depicts an exemplary alternative proximal clevis (418) that is substantially similar to proximal clevis (318) described above. As will be understood, proximal clevis (418) can be readily incorporated into electrosurgical instrument (300) or other suitable surgical tools or instruments in lieu of proximal clevis (318) described above. Although the term "proximal clevis" to refer to proximal clevis (418) is used herein, in some contexts the terms "coupling member," "coupler," or simply "clevis" may be used to refer to proximal clevis (418). As with proximal clevis (318) discussed above, proximal clevis (418) of the present example is configured to fasten to the distal end of shaft (302) and extend distally therefrom. Similarly, proximal clevis (418) is generally configured to receive a portion of distal clevis (316) to permit rotation of distal clevis (316) relative to shaft (302). In other words, proximal clevis (418) provides a point of connection between distal clevis (316) and shaft (302) to promote movement of distal clevis (316) relative to shaft (302). As similarly noted above, distal clevis (316) may be fastened to proximal clevis (418) by pin (324), which defines the pitch axis for rotation of distal clevis (316). Additionally, proximal clevis (418) is generally hollow such that cables (308, 310, 312, 314) and/or one or more wires (319) may extend through proximal clevis (418) to distal clevis (316) and/or end effector (304). To support the function discussed above, proximal clevis (418) may include certain structural features such as an annular base having an irregular shaped central opening. In some examples, proximal clevis (418) may further include a pair of arms extending distally from the annular base to support pin (324) and distal clevis (316) as noted above.

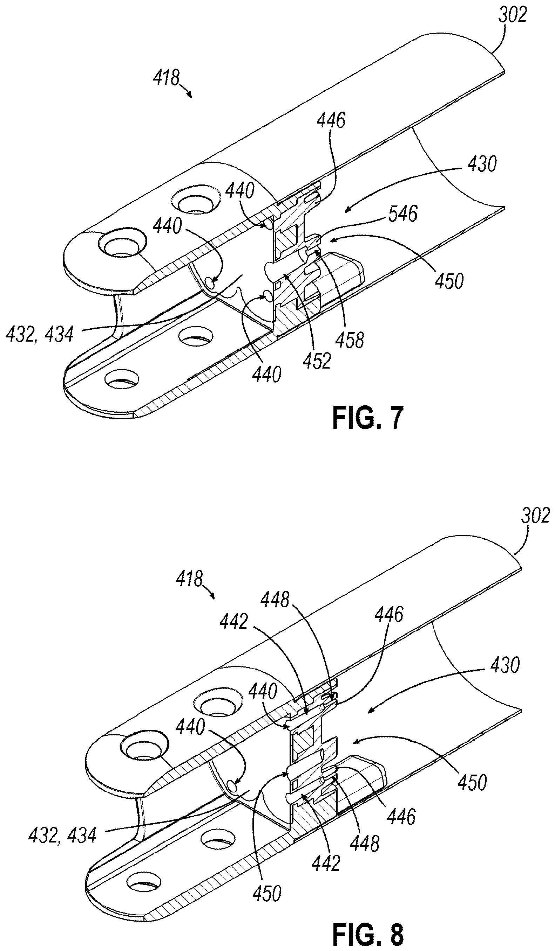

As best seen in FIGS. 7 and 8, unlike proximal clevis (318) discussed above, proximal clevis (418) of the present example includes a sealing feature (430) disposed within an interior portion of clevis (418). Sealing feature (430) is generally configured to provide a robust fluid tight seal at the distal end of shaft (302) to prevent fluid ingress (e.g., liquid and gas ingress) into shaft (302) from the exterior of shaft (302). Meanwhile, sealing feature (430) is also generally configured to permit various components such as cables and/or wires to readily communicate with end effector (304) while maintaining the robust fluid seal noted above. As will be discussed in greater detail below, such communication with end effector (304) through sealing feature (430) may include movement of various components relative to sealing feature (430). Thus, sealing feature (430) includes certain features to promote movement of components relative thereto, while also maintaining a fluid tight seal. Although sealing feature (430) is discussed herein in connection with clevis (418), it should be understood that in other examples, sealing feature (430) can be readily incorporated into other components of tool (300) or other tools or instruments entirely.

Sealing feature (430) of the present example is generally formed of a unitary compliant material overmolded into the interior of clevis (418). Use of a compliant material for sealing feature (430) is generally desirable to promote reduced wear. Use of a compliant material for sealing feature (430) is further desirable to provide flexion of sealing feature (430) while maintaining a sufficient seal with elongate components extending through sealing feature (430). In some examples, suitable compliant materials may include silicone or other similar materials. Although sealing feature (430) is described herein as being overmolded into proximal clevis (418), it should be understood that in some examples, sealing feature (430) may alternatively be molded separately and then attached to proximal clevis (418) by adhesive bonding, welding, and/or mechanical fastening.

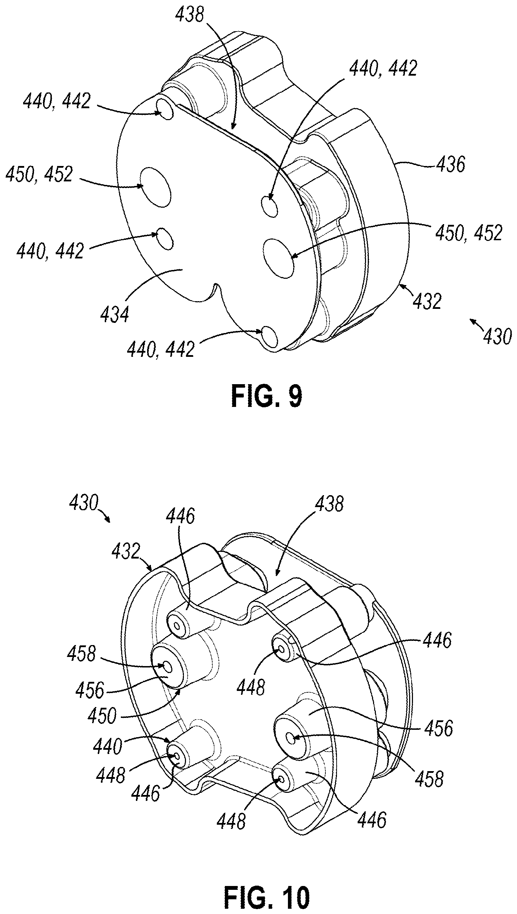

As best seen in FIGS. 7-10, sealing feature (430) includes a seal body (432), and a plurality of projections, referred to herein as dimples, (440, 450) that extend axially relative to seal body (430). Seal body (432) generally extends transversely across the hollow interior defined by proximal clevis (418) to seal the interior of shaft (302) relative to the exterior of shaft (302). As will be described in greater detail below, aside from dimples (440, 450), seal body (432) is generally continuous across the hollow interior of proximal clevis (418) to prevent the flow of fluid therethrough.

Seal body (432) is generally configured to conform to the interior structure of proximal clevis (418). In the present example, seal body (432) has a generally irregular shape that in some ways may be characterized as a multi-part structure with a distal sealing portion (434), a proximal sealing portion (436), and a gap (438) positioned therebetween. In some examples, such a structure of seal body (432) can be formed by at least a portion of proximal clevis (418) to provide additional structural rigidity to seal body (432). For instance, gap (438) may be formed by an internal structure, or structures, of proximal clevis (418) extending through the hollow interior of proximal clevis (418). During the overmolding process, such internal structure or structures may be surrounded with material forming seal body (432).

Although a specific structure for seal body (432) is shown for the present example, it should be understood that seal body (432) may take on a variety of forms depending on the internal structure of proximal clevis (418). Suitable forms for seal body (432) and/or proximal clevis (418) may be configured in accordance with a variety of considerations such as the desired rigidity of seal body (432), ease of manufacturability, the desired surface area of contact between seal body (432) and proximal clevis (418), and/or etc.

As noted above, sealing feature (430) is configured to seal the interior of proximal clevis (418) while still permitting communication of various components with end effector (304). Thus, sealing feature (430) is configured to receive one or more components of tool (300) so that such components may pass through sealing feature (430) to end effector (304). To promote such functionality, sealing feature (430) includes dimples (440, 450) positioned to receive one or more components of tool (300) as will be described in greater detail below.

As can be seen, each dimple (440, 450) extends both proximally within seal body (432) from distal sealing portion (434) and proximally from proximal sealing portion (436) of seal body (432). Accordingly, each dimple (440, 450) defines a generally cone-shaped protrusion through which certain portions of tool (300) may extend. Although each dimple (440, 450) is shown in the present example as projecting proximally, it should be understood that in other examples, one or more of dimples (440, 450) can alternatively project distally rather than proximally. In such configurations, the various structures of dimples (440, 450) described herein would be generally reversed from proximal to distal or distal to proximal.

Each dimple (440, 450) includes a tapered receiving portion (442, 452) and a sealing portion (446, 456). Receiving portion (442, 452) of each dimple (440, 450) is defined by seal body (432) as a bore extending through seal body (432). The shape of receiving portion (442, 452) of each dimple (440, 450) is generally cylindrical and tapered, similar to a countersink. By way of example only, in some instances the taper angle of each receiving portion (442, 452) may be about 8°.

Sealing portion (446, 456) of each dimple (440, 450) projects proximally from a corresponding receiving portion (442, 452). Additionally, each sealing portion (446, 456) projects proximally from a proximal face of seal body (432). As will be described in greater detail below, each sealing portion (446, 456) may be configured to bend or flex to maintain a fluid-tight seal in response to movement of one or more components of tool (300).

Sealing portion (446, 456) of each dimple (440, 450) includes a sealing bore (448, 458) extending longitudinally through the entirety of sealing portion (446, 456). Each sealing bore (448, 458) is in communication with a corresponding receiving portion (442, 452) to define a continuous longitudinal path through the entirety of sealing feature (430). As will be described in greater detail below, this continuous longitudinal path generally permits various elongate components of tool (300) to pass through sealing feature (430).

Each sealing bore (448, 458) of the present example defines a generally cylindrical shape. The particular diameter of each sealing bore (448, 458) generally corresponds to the diameter of a particular component of tool (300) as will be described in greater detail below. Regardless of the particular component received within each sealing bore (448, 458), it should be understood that the diameter of each sealing bore (448, 458) is configured to provide a sealing fit with the particular component received therein. In some examples, this sealing fit may be characterized as a compression or interference fit. Additionally, such a sealing fit may still be configured to provide at least some translation movement of corresponding components of tool (300) relative to each sealing portion (446, 456).

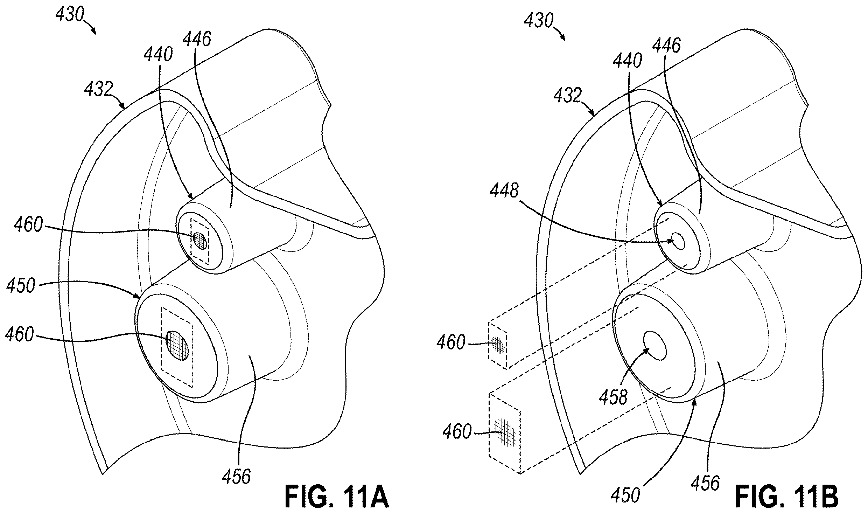

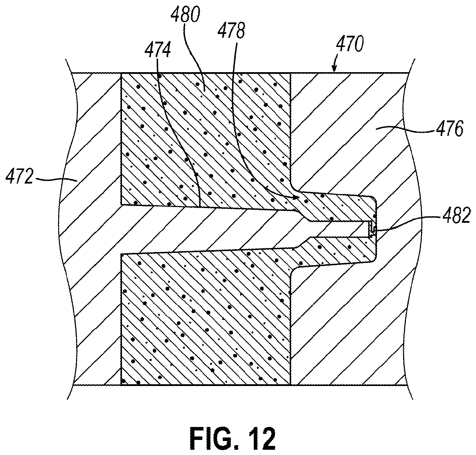

FIGS. 11A through 12 show an exemplary process for forming each sealing bore (448, 458) during the overmolding process noted above. In some overmolding processes, a first mold portion (472) of a mold (470) may include a core pin (474), pin, or other structure may be used to form through holes similar to each sealing bore (448, 458). However, in some versions of the present example, the scale of each sealing bore (448, 458) could lead to core pin (474) or other similar structures contacting an opposing surface of a second mold portion (476) of mold (470) used in the overmolding process. This is a generally undesirable condition because it may result in damage to core pin (474) or other portions of mold (470). Thus, in some overmolding processes, it may be desirable to include steps or features to avoid contact between at least a free end of core pin (474) and other portions of mold (470).

In the present exemplary process, each core pin (474) of mold (470) is shortened such that only a portion of each sealing bore (448, 458) is formed by the respective core pin (474) itself. This may result in a proximal end portion of each sealing bore (448, 458) being capped or filled with a thin layer of excess material or web (460), where web (460) is formed integrally with the proximal end of the respective dimple (440, 450) and thus is disposed opposite to and in confronting relation with the free end of the respective core pin (474) during the overmolding process. Each web (460) may later be removed to form a proximal end opening of each sealing bore (448, 458) after the molding process is complete.

Once the molding process is complete, each web (460) can be removed from the respective dimple (440, 450) using a variety of processes. For instance, in one exemplary process, a screw, hook, or other gripping feature (482) may be integrated into second mold portion (476) of mold (470) or a similar portion thereof that opposes the particular mold portion containing core pins (474) and at locations aligned with each dimple (440, 450) being formed such that a free end of each gripping feature projects toward and confronts a free end of the corresponding core pin (474). Such a screw, hook, or gripping feature may engage web (460) during molding , for example by being partially embedded within web (460), and subsequently tear web (460) along with any other excess material away from the respective formed dimple (440, 450) when the mold portions (472, 476) are separated from one another after molding is complete. To promote such tearing of each web (460) without imparting unintended deformation to the sealing portion (446, 456) of the corresponding formed dimple (440, 450), web (460) may be formed with a thickness in an axial direction that is less than the wall thickness of the sealing portion (446, 456) in a radial direction. In other examples, web (460) and any excess material can be removed from the proximal end portion of each formed dimple (440, 450) via piercing with a sharp object. Alternatively, in still other examples, web (460) and any excess material can be removed using a laser cutting process. Still other alternative processes for removal of web (460) and any excess materials will be apparent to those of ordinary skill in the art in view of the teachings herein.

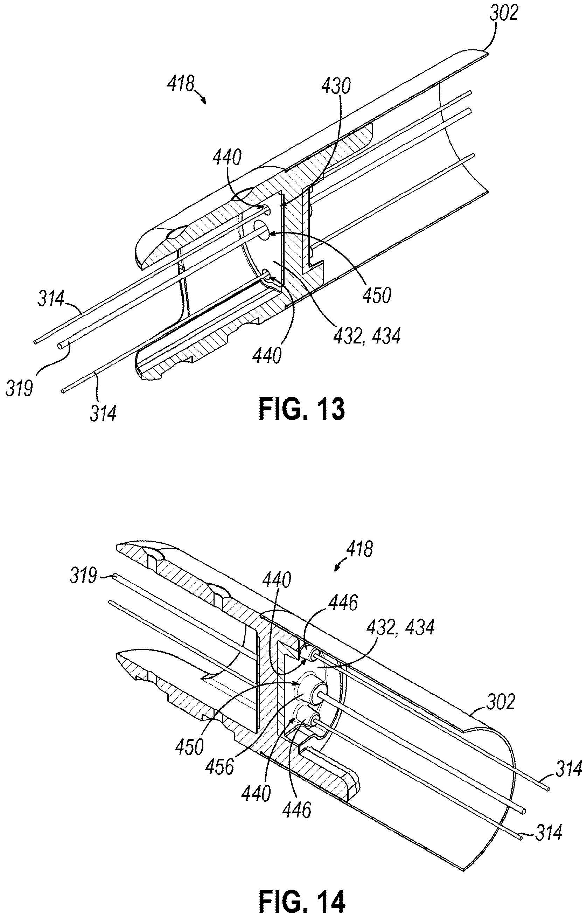

Dimples (440, 450) of the present example may be configured to receive differently sized components of tool (300). For instance, as best seen in FIGS. 13 and 14, sealing feature (430) of the present example includes four cable dimples (440) configured to receive cables (308, 310, 312, 314), respectively, described above. As such, sealing bore (448) of each cable dimple (440) has a diameter corresponding to the outer diameter of a corresponding cable (308, 310, 312, 314) such that sealing portion (446) of each cable dimple (440) is configured to sealingly engage a corresponding cable (308, 310, 312, 314). Similarly, the particular number of cable dimples (440) corresponds to the particular number of cables (308, 310, 312, 314). Thus, in examples where tool (300) includes more or less cables (308, 310, 312, 314), sealing feature (430) may likewise include more or less cable dimples (440).

Sealing feature (430) of the present example also includes two wire dimples (450) configured to receive respective wires (319) described above. As such, sealing bore (458) of each wire dimple (450) has a diameter corresponding to the outer diameter of a corresponding wire (319) such that sealing portion (456) of each wire dimple (450) is configured to sealingly engage a corresponding wire (319). Similarly, the particular number of each wire dimple (450) corresponds to the particular number of wires (319). Thus, in examples where tool (300) includes more or less wires (319), sealing feature (430) may likewise include more or less wire dimples (450).

Although dimples (440, 450) are described herein in the context of receiving either cables (308, 310, 312, 314) or wires (319), it should be understood that in other examples, dimples (440, 450) may be configured to readily receive other components of surgical tool (300). For instance, in some examples tool (300) may include tubes, cannulas, and/or etc. to provide various fluids to end effector (304). In such examples, sealing feature (430) may likewise include structures similar to dimples (440, 450) for receiving such tubes, cannulas, and/or etc.

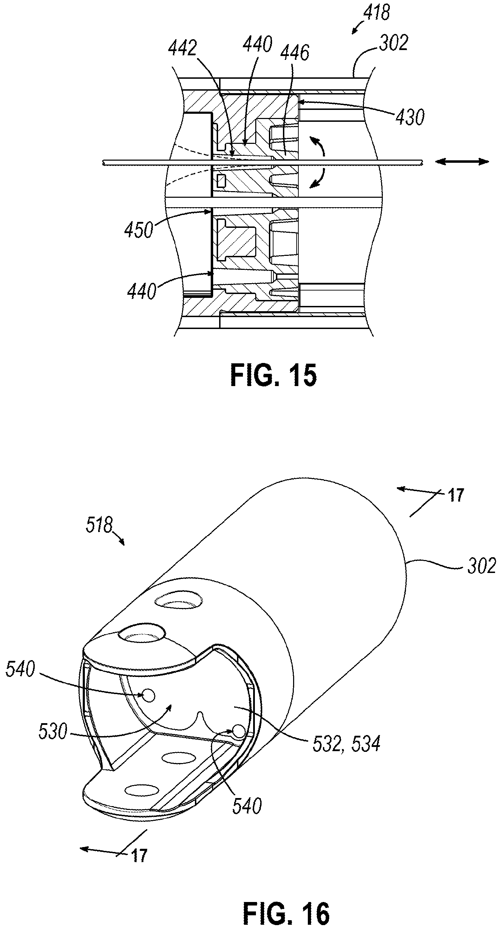

As best seen in FIG. 15, each cable dimple (440) is configured to receive a corresponding cable (308, 310, 312, 314) such that the corresponding cable (308, 310, 312, 314) remains free to slide and/or translate longitudinally through and relative to sealing feature (430). Additionally, each cable dimple (440) is configured to transversely flex relative to a longitudinal axis of shaft (302) to accommodate transverse movement of cables (308, 310, 312, 314). Such transverse flexion may be desirable to accommodate articulation of end effector (304). For instance, in use, articulation of end effector (304) may result in some deflection of cables (308, 310, 312, 314) during the course of articulation. With each cable dimple (440) flexing in response to such deflection of cables (308, 310, 312, 314), each cable dimple (440) is configured to maintain sealing engagement with each corresponding cable (308, 310, 312, 314) despite such deflection. Although not shown in FIG. 15, it should be understood that wire dimples (450) may likewise support such deflection of wires (319) during articulation of end effector (304).

FIG. 15 depicts another exemplary alternative proximal clevis (518) that is substantially similar to proximal clevis (318) described above. As will be understood, proximal clevis (518) can be readily incorporated into electrosurgical instrument (300) or other suitable surgical tools or instruments in lieu of proximal clevis (318) described above. As with proximal clevis (318) discussed above, proximal clevis (518) of the present example is configured to fasten to the distal end of shaft (302) and extend distally therefrom. Similarly, proximal clevis (518) is generally configured to receive a portion of distal clevis (316) to permit rotation of distal clevis (316) relative to shaft (302). In other words, proximal clevis (518) provides a point of connection between distal clevis (316) and shaft (302) to promote movement of distal clevis (316) relative to shaft (302). As similarly noted above, distal clevis (316) may be fastened to proximal clevis (518) by pin (324), which defines the pitch axis for rotation of distal clevis (316). Additionally, proximal clevis (518) is generally hollow such that cables (308, 310, 312, 314) and/or one or more wires (319) may extend through proximal clevis (518) to distal clevis (316) and/or end effector (304).

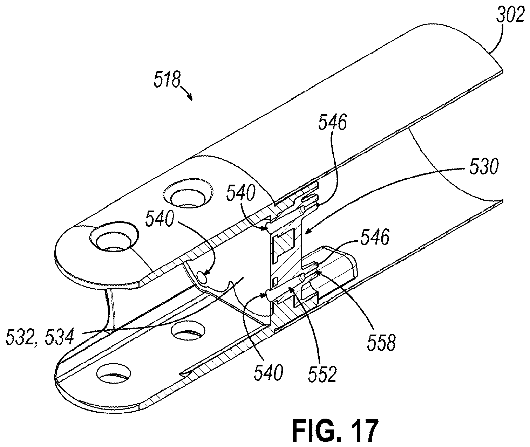

As best seen in FIG. 17, unlike proximal clevis (318) discussed above, proximal clevis (518) of the present example includes a sealing feature (530) disposed within an interior portion of clevis (518). Sealing feature (530) is generally configured to provide a robust fluid tight seal at the distal end of shaft (302) to prevent fluid ingress into shaft (302) from the exterior of shaft (302). Meanwhile, sealing feature (530) is also generally configured to permit various components such as cables (308, 310, 312, 314) and/or wires (319) to readily communicate with end effector (304) while maintaining the robust fluid seal noted above. As will be discussed in greater detail below, such communication with end effector (304) through sealing feature (530) may include movement of various components relative to sealing feature (530). Thus, sealing feature (530) includes certain components to promote movement of components relative thereto, while also maintaining a fluid tight seal. Although sealing feature (530) is discussed herein in connection with clevis (518), it should be understood that in other examples, sealing feature (530) can be readily incorporated into other components of tool (300) or other tools and/or instruments entirely.

Sealing feature (530) of the present example is generally substantially similar to sealing feature (430) described above. For instance, like sealing feature (430), sealing feature of the present example is formed of a unitary compliant material overmolded or otherwise fastened into the interior of proximal clevis (518). Sealing feature (530) also includes a seal body (532), similar to seal body (432), defining a plurality of dimples (540). As similarly discussed above, seal body (532) generally extends transversely across the hollow interior defined by proximal clevis (518) to seal the interior of shaft (302) relative to the exterior of shaft (302).

Like with seal body (432) described above, seal body (532) of the present example has a generally irregular shape defining a distal sealing portion (534), a proximal sealing portion (536), and a gap (538) positioned therebetween. In some examples, such a structure of seal body (532) can be formed by at least a portion of proximal clevis (518) to provide additional structural rigidity to seal body (532). For instance, gap (538) may be formed by an internal structure, or structures, of proximal clevis (518) extending through the hollow interior of proximal clevis (518). During the overmolding process, such internal structure or structures may be surrounded with material forming seal body (532).

As also with sealing feature (430) described above, sealing feature (530) of the present example includes dimples (540) positioned to receive one or more components of tool (300). As similarly discussed above, each dimple (540) extends both proximally within seal body (532) from distal sealing portion (534) and proximally from proximal sealing portion (536) of seal body (532). Accordingly, each dimple (540) defines a generally cone-shaped protrusion through which certain portions of tool (300) may extend. Although each dimple (540) is shown in the present example as projecting proximally, it should be understood that in other examples, one or more of dimples (540) can alternatively project distally rather than proximally. In such configurations, the various structures of dimples (540) described herein would be generally reversed from proximal to distal or distal to proximal.

As with dimples (440) described above, each dimple (540) includes a tapered receiving portion (542) and sealing portion (546). Receiving portion (542) of each dimple (540) is defined by seal body (532) as a tapered bore extending through seal body (532). Meanwhile, sealing portion (546) of each dimple (540) projects proximally from a corresponding receiving portion (542) and also projects proximally from a proximal face of seal body (532). Sealing portion (546) of each dimple (540) also includes a sealing bore (548), similar to sealing bore (448), extending longitudinally through the entirety of sealing portion (546). As with sealing bore (448) described above, each sealing bore (548) of the present example defines a generally cylindrical shape generally corresponds to the diameter of a particular component of tool (300) therein to provide a sealing fit with such a component of tool (300).

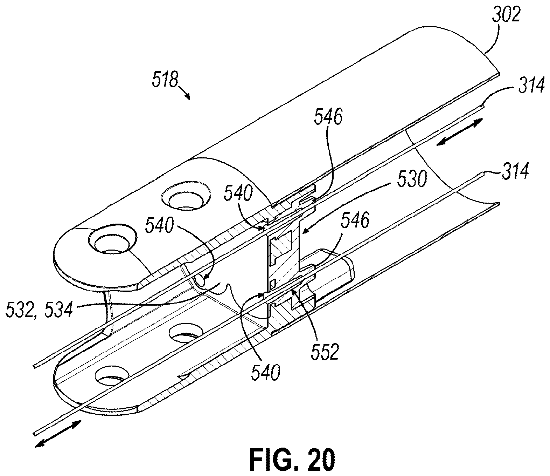

Dimples (540) of the present example may be configured to receive differently sized components of tool (300). For instance, as best seen in FIG. 20, sealing feature (530) of the present example includes four cable dimples (540) configured to receive cables (308, 310, 312, 314) described above. As such, sealing bore (548) of each cable dimple (540) has a diameter corresponding to the outer diameter of a corresponding cable (308, 310, 312, 314) such that sealing portion (546) of each cable dimple (540) is configured to sealingly engage a corresponding cable (308, 310, 312, 314). Similarly, the particular number of each cable dimple (540) corresponds to the particular number of cables (308, 310, 312, 314). Thus, in examples where tool (300) includes more or less cables (308, 310, 312, 314), sealing feature (530) may likewise include more or less cable dimples (540).

Unlike sealing feature (430) described above, sealing feature (530) of the present example omits structures similar to wire dimples (450). Thus, sealing feature (540) of the present example is configured for use in version of tool (300) without one or more wires (319), or versions of tool (300) where wires (319) are present, but do not extend all the way to end effector (304).

Although dimples (540) are described herein in the context of receiving cables (308, 310, 312, 314), it should be understood that in other examples, dimples (540) may be configured to readily receive other components. For instance, in some examples tool (300) may include tubes, cannulas, and/or etc. to provide various fluids to end effector (304). In such examples, sealing feature (530) may likewise include structures similar to dimples (540) for receiving such tubes, cannulas, and/or etc.

As best seen in FIG. 20, each cable dimple (540) is configured to receive a corresponding cable (308, 310, 312, 314) such that the corresponding cable (308, 310, 312, 314) remains free to slide and/or translate relative to sealing feature (530). Additionally, each cable dimple (540) is configured to transversely flex to accommodate transverse movement of cables (308, 310, 312, 314). Such transverse flexion may be desirable to accommodate articulation of end effector (304). For instance, in use, articulation of end effector (304) may result in some deflection of cables (308, 310, 312, 314) during the course of articulation. With each cable dimple (540) flexing in response to such deflection of cables (308, 310, 312, 314), each cable dimple (540) is configured to maintain sealing engagement with each corresponding cable (308, 310, 312, 314) despite such deflection.

### V. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

## Claims

1. A surgical apparatus (100, 300) , comprising
(a) a body (110);
(b) a shaft assembly (102,302) extending distally from the body, wherein the shaft assembly includes a distal end;
(c) an end effector (104);
(d) a coupling member (418) disposed at the distal end of the shaft assembly to movably couple the end effector to the shaft assembly; and
(e) a seal feature (430) engaged with the coupling member, wherein the seal feature includes a seal body (432) and a plurality of protrusions (440, 450) extending from the seal body, wherein each protrusion of the plurality of protrusions is configured to slidably receive a respective elongate member associated with the end effector therethrough, **characterised in that** the seal feature is integrated into the structure of the coupling member.

2. The apparatus of claim 1, wherein each protrusion defines a receiving portion (442, 452) and a sealing portion (446, 456), wherein the sealing portion is configured to sealingly engage the elongate member.

3. The apparatus of claim 2, wherein the sealing portion is configured to flex in response to transverse movement of the elongate member relative to a longitudinal axis of the shaft assembly.

4. The apparatus of claim 2, wherein the sealing portion is configured to flex in response to transverse movement of the elongate member relative to a longitudinal axis of the shaft assembly while maintaining sealing engagement with the elongate member.

5. The apparatus of claim **1,** wherein the sealing portion defines a seal bore (448, 458), wherein the seal bore defines a diameter that is approximately equal to a diameter of the elongate member.

6. The apparatus of claim **1,** wherein the receiving portion defines a tapered bore extending therethrough and in communication with at least a portion of the sealing portion.

7. The apparatus of claim **1,** further comprising a plurality of cables (308, 310, 312, 314) extending from the body to the end effector through the shaft assembly, wherein each cable is movable relative to the body to drive movement of the end effector, wherein each protrusion of the plurality of protrusions is configured to slidably receive a corresponding cable to permit movement of the end effector via one or more cables of the plurality of cables.

8. The apparatus of claim 7, wherein each protrusion is configured to transversely flex in response to transverse deflection of a corresponding cable via movement of the end effector.

9. The apparatus of claim **1,** further comprising a plurality of cables configured to drive the end effector and one or more wires (319) configured to communicate RF energy to the end effector, wherein the plurality of cables and the one or more wires extend from the body to the end effector through the shaft assembly, wherein one or more protrusions of the plurality of protrusions is configured to slidably receive a cable of the plurality of cables, wherein one or more protrusions of the plurality of protrusions (450) is configured to slidably receive a wire of the one or more wires.

10. The apparatus of claim 9, wherein each protrusion of the plurality of protrusions is configured to flex in response to transverse movement of a respective cable or respective wire.

11. The apparatus of claim **1,** wherein each protrusion of the plurality of protrusions extends proximally from the seal body.

12. The apparatus of claim 1, wherein the seal feature comprises a compliant material.

13. The apparatus of claim 12, wherein the seal feature comprises silicone.

14. The apparatus of claim 1, wherein the seal body includes a distal portion and a proximal portion with a gap defined between the proximal portion and the distal portion, wherein a portion of the coupling member extends through the gap between the proximal portion and the distal portion.

15. A method of overmolding a seal feature (430) onto a clevis (418) configured for use with a surgical instrument (100, 300), the method comprising:
(a) positioning the clevis within a mold (470) having a first mold portion (472) and a second mold portion (476), wherein the first mold portion includes a pin (474) having a free end that extends toward and is spaced apart from an opposing surface of the second mold portion;
(b) directing material into the mold and about the pin to thereby form the seal feature from the material such that the pin defines a sealing bore of the seal feature, wherein the seal feature includes a layer of the material between the free end of the pin and the opposing surface of the second mold portion such that the layer of material is at an end of the sealing bore;
(c) after the material has set, removing the layer of material from the seal feature to form an opening to the sealing bore; and
(d) coupling the clevis having the seal feature to a distal end of a shaft assembly of a surgical instrument.

16. The method of claim 15, wherein the second mold portion includes a gripping projection (482) that extends toward and confronts the free end of the pin of the first mold portion, wherein the step of removing the layer of material is performed by separating the first mold portion from the second mold portion such that the gripping projection tears the layer of material away from the seal feature.

## Patentansprüche

1. Chirurgische Einrichtung (100, 300), umfassend
(a) einen Körper (110);
(b) eine Wellenanordnung (102,302), die sich von dem Körper distal erstreckt, wobei die Wellenanordnung ein distales Ende einschließt;
(c) einen Endeffektor (104);
(d) ein Kopplungselement (418), das an dem distalen Ende der Wellenanordnung eingerichtet ist, um den Endeffektor mit der Wellenanordnung bewegbar zu koppeln; und
(e) ein Dichtungsmerkmal (430), das mit dem Kopplungselement in Eingriff steht, wobei das Dichtungsmerkmal einen Dichtungskörper (432) und eine Vielzahl von Vorsprüngen (440, 450) einschließt, die sich von dem Dichtungskörper erstreckt, wobei jeder Vorsprung der Vielzahl von Vorsprüngen konfiguriert ist, um ein jeweiliges längliches Element, das mit dem Endeffektor verknüpft ist, dahindurch gleitend aufzunehmen,
**dadurch gekennzeichnet, dass** das Dichtungsmerkmal in die Struktur des Kopplungselements integriert ist.

2. Einrichtung nach Anspruch 1, wobei jeder Vorsprung einen Aufnahmeabschnitt (442, 452) und einen dichtenden Abschnitt (446, 456) definiert, wobei der dichtende Abschnitt konfiguriert ist, um mit dem länglichen Element dichtend in Eingriff zu stehen.

3. Einrichtung nach Anspruch 2, wobei der dichtende Abschnitt konfiguriert ist, um sich als Reaktion auf eine Querbewegung des länglichen Elements relativ zu einer Längsachse der Wellenanordnung zu biegen.

4. Einrichtung nach Anspruch 2, wobei der dichtende Abschnitt konfiguriert ist, um sich als Reaktion auf die Querbewegung des länglichen Elements relativ zu einer Längsachse der Wellenanordnung zu biegen, während der dichtende Eingriff mit dem länglichen Element aufrechterhalten wird.

5. Einrichtung nach Anspruch **1,** wobei der dichtende Abschnitt eine Dichtungsbohrung (448, 458) definiert, wobei die Dichtungsbohrung einen Durchmesser definiert, der ungefähr gleich einem Durchmesser des länglichen Elements ist.

6. Einrichtung nach Anspruch **1,** wobei der Aufnahmeabschnitt eine konische Bohrung definiert, die sich dahindurch erstreckt, die mit mindestens einem Abschnitt des dichtenden Abschnitts in Kommunikation steht.

7. Einrichtung nach Anspruch **1,** ferner umfassend eine Vielzahl von Kabeln (308, 310, 312, 314), die sich von dem Körper durch die Wellenanordnung hindurch zu dem Endeffektor erstreckt, wobei jedes Kabel relativ zu dem Körper bewegbar ist, um eine Bewegung des Endeffektors anzutreiben, wobei jeder Vorsprung der Vielzahl von Vorsprüngen konfiguriert ist, um ein entsprechendes Kabel verschiebbar aufzunehmen, um die Bewegung des Endeffektors über ein oder mehrere Kabel der Vielzahl von Kabeln zu ermöglichen.

8. Einrichtung nach Anspruch 7, wobei jeder Vorsprung konfiguriert ist, um sich als Reaktion auf eine Querdurchbiegung eines entsprechenden Kabels über die Bewegung des Endeffektors quer zu biegen.

9. Einrichtung nach Anspruch 1, ferner umfassend eine Vielzahl von Kabeln, die konfiguriert ist, um den Endeffektor anzutreiben, und einen oder mehrere Drähte (319), die konfiguriert sind, um HF-Energie an den Endeffektor zu kommunizieren, wobei die Vielzahl von Kabeln und der eine oder die mehreren Drähte sich von dem Körper durch die Wellenanordnung hindurch zu dem Endeffektor erstrecken, wobei ein oder mehrere Vorsprünge der Vielzahl von Vorsprüngen konfiguriert sind, ein Kabel der Vielzahl von Kabeln verschiebbar aufzunehmen, wobei ein oder mehrere Vorsprünge der Vielzahl von Vorsprüngen (450) konfiguriert sind, um einen Draht der einen oder mehreren Drähte verschiebbar aufzunehmen.

10. Einrichtung nach Anspruch 9, wobei jeder Vorsprung der Vielzahl von Vorsprüngen konfiguriert ist, um sich als Reaktion auf die Querbewegung eines jeweiligen Kabels oder Drahts zu biegen.

11. Einrichtung nach Anspruch **1,** wobei sich jeder Vorsprung der Vielzahl von Vorsprüngen von dem Dichtungskörper proximal erstreckt.

12. Einrichtung nach Anspruch 1, wobei das Dichtungsmerkmal ein nachgiebiges Material umfasst.

13. Einrichtung nach Anspruch 12, wobei das Dichtungsmerkmal Silikon umfasst.

14. Einrichtung nach Anspruch 1, wobei der Dichtungskörper einen distalen Abschnitt und einen proximalen Abschnitt einschließt, wobei zwischen dem proximalen Abschnitt und dem distalen Abschnitt ein Spalt definiert ist, wobei sich ein Abschnitt des Kopplungselements durch den Spalt hindurch zwischen dem proximalen Abschnitt und dem distalen Abschnitt erstreckt.

15. Verfahren zum Umspritzen eines Dichtungsmerkmals (430) auf einen Gabelkopf (418), das für eine Verwendung mit einem chirurgischen Instrument (100, 300) konfiguriert ist, das Verfahren umfassend:
(a) Positionieren des Gabelkopfs innerhalb einer Form (470), die einen ersten Formabschnitt (472) und einen zweiten Formabschnitt (476) aufweist, wobei der erste Formabschnitt einen Stift (474) einschließt, der ein freies Ende aufweist, das sich zu einer gegenüberliegenden Oberfläche des zweiten Formabschnitts hin erstreckt und von dieser beabstandet ist;
(b) Leiten von Material in die Form und um den Stift herum, wobei dadurch das Dichtungsmerkmal aus dem Material derart ausgebildet wird, dass der Stift eine dichtende Bohrung des Dichtungsmerkmals definiert, wobei das Dichtungsmerkmal eine Schicht des Materials zwischen dem freien Ende des Stifts und der gegenüberliegenden Oberfläche des zweiten Formabschnitts derart einschließt, dass sich die Materialschicht an einem Ende der dichtende Bohrung befindet;
(c) nachdem das Material ausgehärtet ist, Entfernen der Materialschicht von dem Dichtungsmerkmal, um eine Öffnung zu der dichtende Bohrung auszubilden; und
(d) Koppeln des Gabelkopfs, der das Dichtungsmerkmal aufweist, mit einem distalen Ende einer Wellenanordnung eines chirurgischen Instruments.

16. Verfahren nach Anspruch 15, wobei der zweite Formabschnitt einen Greifvorsprung (482) einschließt, der sich zu dem freien Ende des Stifts des ersten Formabschnitts hin erstreckt und diesem gegenüberliegt, wobei der Schritt des Entfernens der Materialschicht durch Trennen des ersten Formabschnitts von dem zweiten Formabschnitt derart durchgeführt wird, dass der Greifvorsprung die Materialschicht von dem Dichtungsmerkmal wegreißt.

## Revendications

1. Appareil chirurgical (100, 300) comprenant :
(a) un corps (110) ;
(b) un ensemble tige (102, 302) s'étendant distalement à partir du corps, dans lequel l'ensemble tige comporte une extrémité distale ;
(c) un effecteur d'extrémité (104) ;
(d) un élément d'accouplement (418) disposé au niveau de l'extrémité distale de l'ensemble tige pour accoupler de manière mobile l'effecteur d'extrémité à l'ensemble tige ; et
(e) une caractéristique d'étanchéité (430) mise en prise avec l'élément d'accouplement, dans lequel la caractéristique d'étanchéité comporte un corps d'étanchéité (432) et une pluralité de protubérances (440, 450) s'étendant à partir du corps d'étanchéité, dans lequel chaque protubérance de la pluralité de protubérances est conçue pour recevoir de manière coulissante un élément allongé respectif associé à l'effecteur d'extrémité à travers celui-ci,
**caractérisé en ce que** la caractéristique d'étanchéité est
intégrée dans la structure de l'élément d'accouplement.

2. Appareil selon la revendication 1, dans lequel chaque protubérance définit une partie de réception (442, 452) et une partie d'étanchéité (446, 456), dans lequel la partie d'étanchéité est conçue pour se mettre en prise de manière étanche avec l'élément allongé.

3. Appareil selon la revendication 2, dans lequel la partie d'étanchéité est conçue pour fléchir en réponse au déplacement transversal de l'élément allongé par rapport à l'axe longitudinal de l'ensemble tige.

4. Appareil selon la revendication 2, dans lequel la partie d'étanchéité est conçue pour fléchir en réponse au déplacement transversal de l'élément allongé par rapport à l'axe longitudinal de l'ensemble tige tout en maintenant la mise en prise d'étanchéité avec l'élément allongé.

5. Appareil selon la revendication **1,** dans lequel la partie d'étanchéité définit un alésage d'étanchéité (448, 458), dans lequel l'alésage d'étanchéité définit un diamètre qui est approximativement égal à un diamètre de l'élément allongé.

6. Appareil selon la revendication **1,** dans lequel la partie réceptrice définit un alésage conique s'étendant à travers celle-ci et en communication avec au moins une partie de la partie d'étanchéité.

7. Appareil selon la revendication **1,** comprenant en outre une pluralité de câbles (308, 310, 312, 314) s'étendant à partir du corps jusqu'à l'effecteur d'extrémité à travers l'ensemble tige, dans lequel chaque câble est mobile par rapport au corps pour entraîner le déplacement de l'effecteur d'extrémité, dans lequel chaque protubérance de la pluralité de protubérances est conçue pour recevoir de manière coulissante un câble correspondant afin de permettre le déplacement de l'effecteur d'extrémité par l'intermédiaire d'un ou plusieurs câbles de la pluralité de câbles.

8. Appareil selon la revendication 7, dans lequel chaque protubérance est conçue pour fléchir transversalement en réponse à la déflexion transversale d'un câble correspondant par l'intermédiaire du déplacement de l'effecteur d'extrémité.

9. Appareil selon la revendication 1, comprenant en outre une pluralité de câbles conçus pour entraîner l'effecteur d'extrémité et un ou plusieurs fils (319) configurés pour communiquer l'énergie RF à l'effecteur d'extrémité, dans lequel la pluralité de câbles et le ou les fils s'étendent à partir du corps jusqu'à l'effecteur d'extrémité à travers l'ensemble tige, dans lequel une ou plusieurs protubérances de la pluralité de protubérances est conçue pour recevoir de manière coulissante un câble de la pluralité de câbles, dans lequel une ou plusieurs protubérances de la pluralité de protubérances (450) est conçue pour recevoir de manière coulissante un fil de l'un ou plusieurs fils.

10. Appareil selon la revendication 9, dans lequel chaque protubérance de la pluralité de protubérances est conçue pour fléchir en réponse au déplacement transversal d'un câble respectif ou d'un fil respectif.

11. Appareil selon la revendication **1,** dans lequel chaque protubérance de la pluralité de protubérances s'étend de manière proximale à partir du corps d'étanchéité.

12. Appareil selon la revendication 1, dans lequel la caractéristique d'étanchéité comprend un matériau conforme.

13. Appareil selon la revendication 12, dans lequel la caractéristique d'étanchéité comprend du silicone.

14. Appareil selon la revendication 1, dans lequel le corps d'étanchéité comporte une partie distale et une partie proximale avec un espace défini entre la partie proximale et la partie distale, dans lequel une partie de l'élément d'accouplement s'étend à travers l'espace entre la partie proximale et la partie distale.

15. Procédé de surmoulage d'une caractéristique d'étanchéité (430) sur un oeillet de suspension (418) conçu pour être utilisé avec un instrument chirurgical (100, 300), le procédé comprenant :
(a) le positionnement de l'œillet de suspension dans un moule (470) ayant une première partie de moule (472) et une seconde partie de moule (476), dans lequel la première partie de moule comporte une broche (474) ayant une extrémité libre s'étend vers une surface opposée de la seconde partie de moule et est espacée de celle-ci ;
(b) la direction du matériau dans le moule et autour de la broche pour former la caractéristique d'étanchéité à partir du matériau de sorte que la broche définisse un alésage d'étanchéité de la caractéristique d'étanchéité, dans lequel la caractéristique d'étanchéité comporte une couche du matériau entre l'extrémité libre de la broche et la surface opposée de la seconde partie du moule de sorte que la couche de matériau se trouve à une extrémité de l'alésage d'étanchéité ;
(c) après la prise du matériau, le retrait de la couche de matériau de la caractéristique d'étanchéité pour former une ouverture vers l'alésage d'étanchéité ; et
(d) l'accouplement de l'oeillet de suspension ayant une caractéristique d'étanchéité à l'extrémité distale d'un ensemble tige d'un instrument chirurgical.

16. Procédé selon la revendication 15, dans lequel la seconde partie du moule comporte une saillie de préhension (482) qui s'étend vers l'extrémité libre de la broche de la première partie du moule, dans lequel l'étape de retrait de la couche de matériau est effectuée en séparant la première partie de moule de la seconde partie de moule de telle sorte que la saillie de préhension arrache la couche de matériau de la caractéristique d'étanchéité.
